Europäisches Patentamt

European Patent Office

Office européen des brevets

(11) Numéro de publication: **0 162 791**
**A1**

## (12) DEMANDE DE BREVET EUROPEEN

(21) Numéro de dépôt: **85420073.0**

(22) Date de dépôt: **25.04.85**

(51) Int. Cl.⁴: **A 61 M 5/24**

(30) Priorité: **26.04.84 FR 8406907**

(43) Date de publication de la demande:
**27.11.85 Bulletin 85/48**

(84) Etats contractants désignés:
**AT BE CH DE GB IT LI LU NL SE**

(71) Demandeur: **Taddei, André**
**32, rue Centrale**
**F-06300 Nice(FR)**

(72) Inventeur: **Taddei, André**
**32, rue Centrale**
**F-06300 Nice(FR)**

(74) Mandataire: **Maisonnier, Jean**
**28 rue Servient**
**F-69003 Lyon(FR)**

(54) Dispositif à seringue d'injection stérile, notamment pour les dentistes.

(57) L'invention concerne une seringue à piqûres, notamment pour les chirurgiens-dentistes.

L'embout ( 13) de l'aiguille (12) a une partie mâle filetée (14) qui s'adapte dans le manchon fileté (2) de la seringue. Le sommet de l'aiguille est ainsi guide et protégé, avant de passer l'orifice (6) sans en toucher les bords. Ensuite, l'opercule de la carpule (8) est perforé et l'injection peut être effectuée.

Application : observation des règles d'aseptie.

FIG 6

La présente invention est relative à un dispositif à seringue d'injection , destinée à être utilisée notamment par les dentistes , pour effectuer des piqûres dans la bouche de leurs patients.

On sait que , pour les chirurgiens-dentistes, la pratique des piqûres est un acte chirurgical très fréquent, notamment pour effectuer des anesthésies  locales . Le dentiste pique une aiguille creuse reliée à une ampoule d'un produit liquide , dont il assure l'injection en actionnant ensuite une seringue de poussée . On sait que , compte tenu des particularités du tissu dans lequel les chirurgiens-dentiste effectuent généralement leurs piqûres , le piston de la seringue est actionné par un système de poussoir et de points d'appui particulièrement robuste , permettant à l'opérateur d'exercer avec la paume de la main , une forte poussée appliquée au liquide de l'ampoule . Cela explique que les seringues de ce type comprennent un porte-ampoule métallique particulièrement robuste , dont le poussoir est profilé suivant une forme anatomique permettant son adaptation à la paume de la main du chirurgien-dentiste .

En fait , compte tenu d'une part de la fréquence de l'acte chirurgical en question , d'autre part des faibles quantités  injectées à chaque piqûre , on constate que le chirurgien-dentiste est souvent amené à utiliser une même aiguille plusieurs fois , et une même ampoule ou carpule , également plusieurs  fois.. Un problème se pose donc dans la pratique quotidienne , en ce qui concerne l'observation des règles de stérilité , lorsque le chirurgien-dentiste change d'aiguille ou de carpule . On constate que la plupart des systèmes actuellement connus ne permettent pas de respecter convenablement les règles d'aseptie .

La  présente invention a pour but d'éviter ces inconvénients , en réalisant un dispositif d'injection . aiguille-carpule-seringue , permettant une observation très stricte et quasi automatique des règles d'aseptie , lorsque le chirurgien-dentiste effectue les opérations courantes de piqûres et d'injection , notamment lorsqu'il désire changer l'aiguille ou la carpule .

Un dispositif de piqûre et d'injection

2

selon l'invention comprend une seringue à poussoir dans le corps de laquelle on introduit une carpule contenant le produit à injecter , alors que l'extrémité filetée de la seringue est susceptible de recevoir par vissage , l'embout d'une aiguille d'injection creuse , et il est caractérisé en ce que l'extrémité filetée de la seringue est constituée par un manchon tubulaire à filetage intérieur , tandis que par ailleurs, l'embout de l'aiguille est un embout mâle , à filetage extérieur , susceptible de venir s'adapter dans celui du manchon de la seringue .

Suivant une autre caractéristique de l'invention , l'aiguille dépasse au-delà de 'embout fileté mâle, d'une longueur inférieure à la distance qui sépare le sommet de cet embout et l'extrémité inférieure perforable de la carpule , lorsque l'embout est présenté devant le manchon fileté de la seringue . Grâce à cette disposition, l'embout porte-aiguille vient se centrer sur l'extrémité du manchon de la seringue , et positionne ainsi pendant le début du vissage ,l' extrémité supérieure de l'aiguille , avant que celle-ci ne vienne perforer la capsule de caoutchouc qui obture la carpule . Cette disposition est importante , car elle évite tout tâtonnement , et notamment tout contact intempestif de l'extrémité supérieure de l'aiguille avec les parois de la seringue , contacts qui sont incompatibles avec le respect des règles d'aseptie.

Le dessin annexé , donné à titre d'exemple non limitatif , permettra de mieux comprendre les caractéristiques de l'invention .

Figure 1 représente une carpule de type connu contenant le liquide à injecter.

Figure 2 montre une aiguille selon l'invention , au moment où on la dégage de son emballage stérile .

Figure 3 montre une seringue selon l'invention ,prête à recevoir la carpule .

Figure 4 montre la seringue au moment où on lui présente l'aiguille .

Figures 5 et 6 sont des coupes longitudinales suivant V - V ( figure 4) , montrant deux phases successives pour la mise en place de l'aiguille.

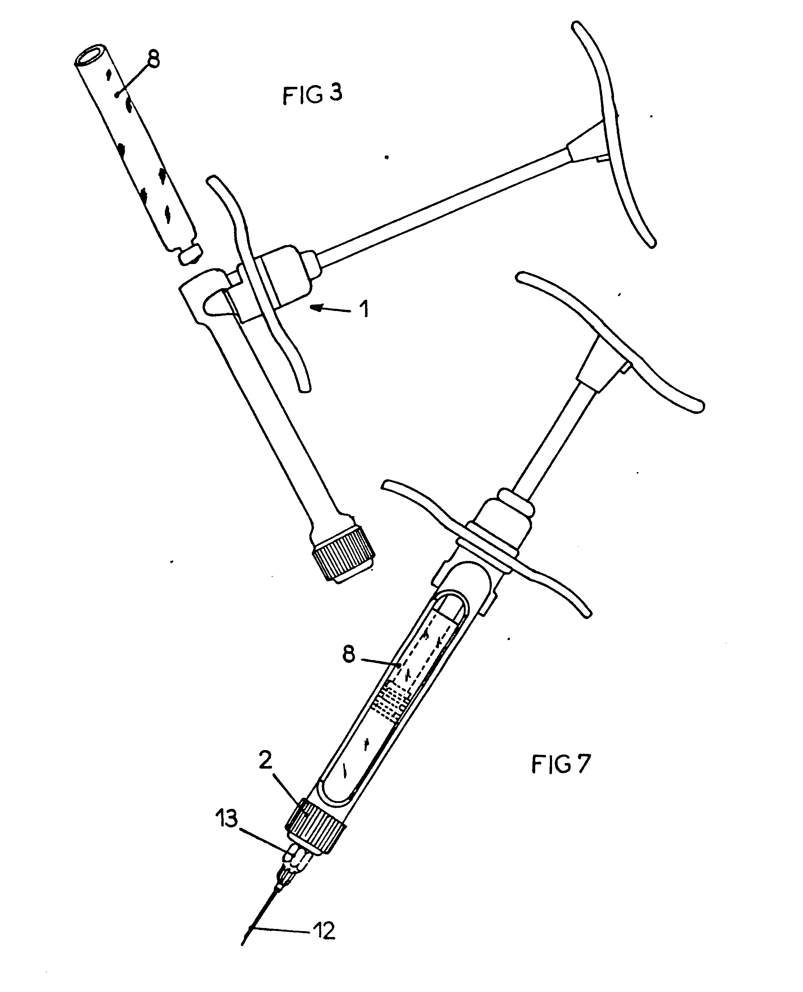

Figure 7 montre le dispositif assemblé , prêt à l'emploi.

Figures 8 à 10 illustrent plusieurs variantes.

On a représenté , sur les dessins, une seringue métallique 1 , dont le type est bien connu auprès des chirurgiens-dentistes . Selon l'invention , l'extrémité inférieure de cette seringue comporte un manchon tubulaire 2 , muni ,sur sa face inférieure , d'un filetage 3.

Pour le reste , le corps 4 de la seringue comporte , à la manière habituelle , un fond 5 , muni d'une perforation centrale 6 , susceptible de servir de butée à la périphérie de l'extrémité 7 d'une carpule 8 de type connu . On sait que cette dernière contient , dans sa partie 9 , le liquide à injecter , tandis que son obturation est assurée par un bochoo intérieur 10 , susceptible de fonctionner à la manière d'un piston à coulissement étanche , lorsqu'il est enfoncé par le poussoir 21 de la seringue.

L'extrémité 7 de la carpule 8 est munie,à la manière connue , d'un opercule obturateur 11, en caoutchouc .

L'ensemble selon l'invention est complété par une aiguille creuse 12 de type connu , solidaire d'un embout en matière plastique 13 qui , selon l'invention , présente la particularité de se prolonger par une partie mâle filetée 14 , susceptible de venir se visser dans le manchon tubulaire 2 de la seringue . L'ensemble de l'aiguille 12 et de son embout 13 est présenté , à la manière connue , dans un emballage stérile , comprenant un tube 15 que referme un bouchon amovible 16 .

Suivant une autre caractéristique préférée de l'invention , l'aiguille 12 dépasse , au-dessus de la partie mâle 14 de l'embout fileté 13 , suivant une distance 17 légèrement inférieure à la distance 19 qui sépare l'extrémité du manchon tubulaire 2 , et la capsule obturatrice 11 de la carpule 8 , lorsqu'elle est en place dans la seringue 1 .

Le fonctionnement est le suivant :

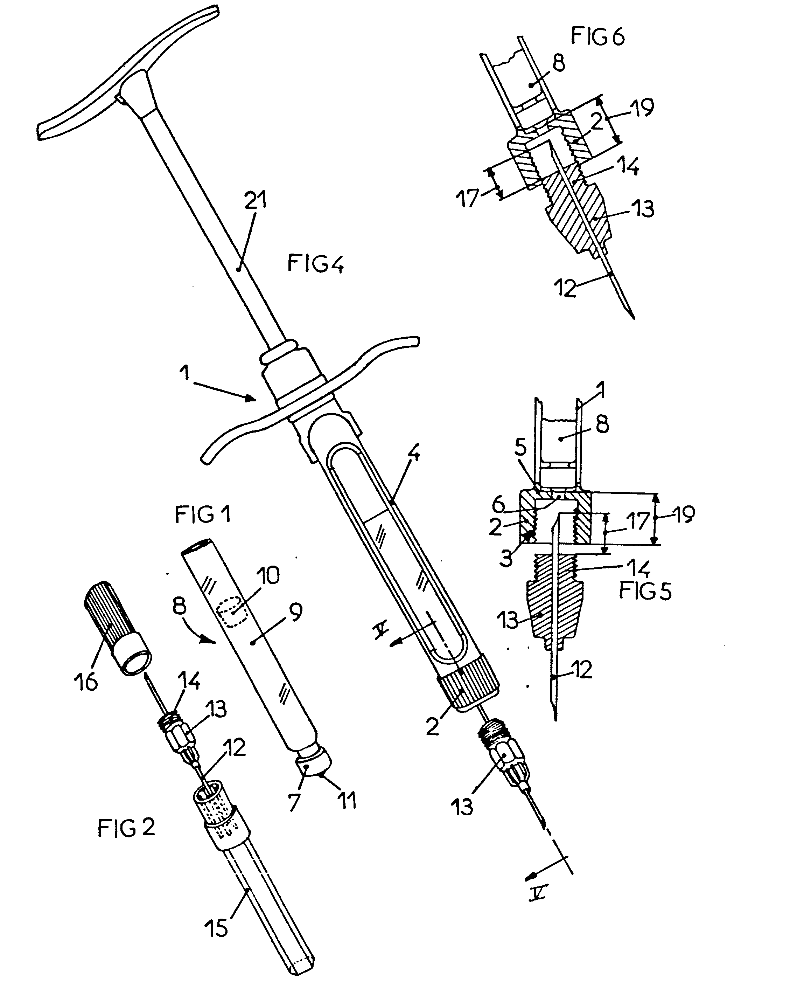

L'utilisateur met en place la carpule 8 dans la seringue 1 , suivant le processus habituel ( figu - re 3) . Une fois la seringue 1 refermée , l'utilisateur ouvre l'emballage 15 , 16 , et il saisit par l'embout 13, l'ensemble de l'aiguille qu'il présente comme illustré sur la figure 4 , devant le manchon 2 de la seringue . Ce faisant , la partie filetée mâle 14 vient se centrer contre l'intérieur fileté 3 du manchon 2 , dans lequel on effectue la fixation par vissage . C'est seulement après le début du vissage , que l'extrémité supérieure de l'aiguille 12 vient en contact avec l'opercule de caoutchouc 6 de la carpule 8, qu'lle perfore ensuite au fur et à mesure que le vissage se termine . On voit que , pendant toute cette opération , l'opérateur n'a touché que l'embout 13 en matière plastique , alors que le centrage de la partie filetée 14 sur le manchon tubulaire 2 interdit à l'extrémité supérieure de l'aiguille 12 d'entrer en contact avec quoi que ce soit avant de perforer l'opercule 6 .

Lorsque l'ensemble est prêt à l'emploi (figure 6) , l'aiguille 12 peut être extraite et remplacée par une autre aiguille , sans que le praticien n'ait à toucher autre chose que l'embout 13 . Inversement , l'aiguille 12 restant en place sur la seringue 1 ( figure 6) , le praticien peut retirer la carpule 8 et la remplacer par une autre , sans enfreindre les règles de l'aseptie.

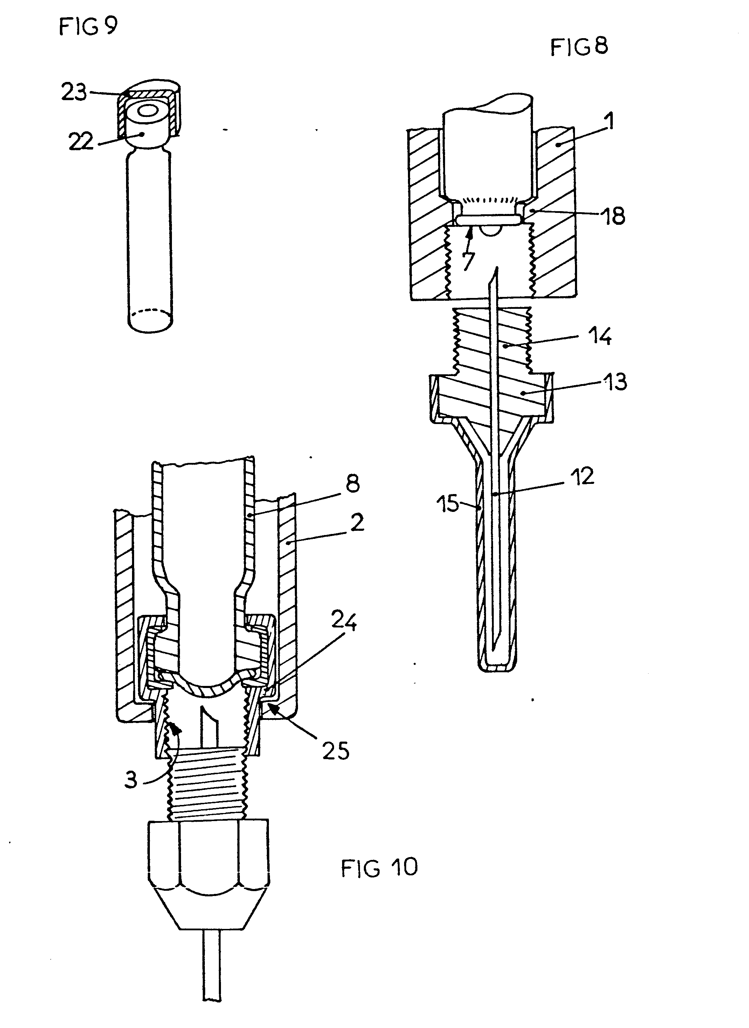

Dans la variante de la figure 8 ,le fond 5 est remplacé par un simple épaulement de butée 18 contre lequel vient porter le col 19 de la carpule 8 .L'extrémité 7 de la carpule 8 est désormais libre sur toute sa surface pour recevoir , en fin de vissage , l'appui de l'extrémité 20 de la partie filetée mâle 14 . cet appui contribue à assurer l'étanchéité à la pression , au moment où le praticien enfonce le poussoir 21 .

La figure 9 montre une variante pour la présentation stérile de la carpule 8 . Son embout d'extrémité 22 est coiffé d'une capsule 23 ou d'une gaine en matière plastique que l'opérateur élimine avant usage .

Sur la variante de la figure 10 , l'embout 22 de la carpule 8 est livré muni d'un manchon inamovible 24 , comportant :

- un épaulement extérieur 25 , pour buter contre l'intérieur de la seringue 2 ;

- le filetage intérieur 3 , pour recevoir la partie mâle filetée 14 de l'aiguille 12 .

Dans ce cas , l'aiguille 12 vient donc se visser directement sur la carpule , la seringue 2 n'étant plus utilisée que pour tenir l'ensemble et refouler le piston 10 .

6
REVENDICATIONS

1 - Dispositif de piqûre et d'injection , comprenant une seringue (1) à poussoir (21) , dans le corps (4) de laquelle on introduit une carpule (8) contenant le produit à injecter , alors que l'extrémité filetée de la seringue est susceptible de recevoir , par vissage , l'embout (13) d'une aiguille d'injection creuse (12) , caractérisé en ce que l'extrémité filetée de la seringue (1) est constituée par un manchon tubulaire (2) , (24) , à filetage intérieur (3) , tandis que , par ailleurs ,l'embout (13) de l'aiguille (12) possède une partie mâle (14) , à filetage extérieur , susceptible de venir s'adapter dans le filetage (3) du manchon (2) , (24) de la seringue (1).

2 - Dispositif selon la revendication 1 , caractérisé en ce que l'aiguille (12) dépasse au-delà de la partie filetée mâle (14) d'une longueur (17) inférieure à la distance (19) qui sépare le sommet de l'embout (13) et l'extrémité inférieure perforable (7) de la carpule (8) , lorsque cet embout (13) est présenté devant le manchon fileté (2) , (24) de la seringue (1).

3 - Dispositif suivant l'une quelconque des revendications précédentes , caractérisé en ce que le manchon (2) à filetage femelle (3) est solidaire du corps (4) de la seringue (1).

4 - Dispositif suivant l'une quelconque des revendications 1 et 2 , caractérisé en ce que le manchon (24) à filetage femelle (3) fait partie de la carpule (8) avec laquelle il est livré .

5 - Dispositif suivant l'une quelconque des revendications précédentes , caractérisé en ce que la carpule (8) est livrée avec son embout (22) , coiffé d'une capsule stérile amovible (23).

0162791

PL.1/3

FIG 6

FIG4

FIG 1

FIG 2

8

FIG 3

1

8

2

13

FIG 7

12

FIG 9

FIG 8

FIG 10

Office européen
des brevets

**RAPPORT DE RECHERCHE EUROPEENNE**

**0162791**
Numéro de la demande

EP 85 42 0073

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int Cl.4) |
|---|---|---|---|
| X | US-A-3 092 108 (FRIEDMAN)<br>* Colonne 4; lignes 16-73; figures * | 1,2,5 | A 61 M 5/24 |
| X | US-A-2 672 867 (ASHKENAZ)<br>* Colonne 2, lignes 21-53; figures * | 1-3 | |
| A | FR-A-2 212 157 (ORILLARD)<br>* Figure 4 * | 4 | |

DOMAINES TECHNIQUES
RECHERCHES (Int. Cl.4)

A 61 M

Le présent rapport de recherche a été établi pour toutes les revendications

| Lieu de la recherche | Date d'achèvement de la recherche | Examinateur |
|---|---|---|
| LA HAYE | 25-07-1985 | VANRUNXT J.M.A. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03 82